# EUROPÄISCHE PATENTSCHRIFT

(11) **EP 0 879 036 B1**
(45) Veröffentlichungstag und Bekanntmachung des Hinweises auf die Patenterteilung: **02.05.2002**
(21) Anmeldenummer: 97901021.2
(22) Anmeldetag: 13.01.1997
(51) Int. Cl.: A61F 13/02, A61B 5/00

(54) **FIXIERVORRICHTUNG FÜR SENSOREN AUF DER HAUTOBERFLÄCHE UND VERFAHREN ZUM NACHWEIS DES ENTFERNENS VON SENSOREN VON DER HAUTOBERFLÄCHE**
DEVICE FOR FASTENING OF SENSORS TO THE SURFACE OF THE SKIN AND A METHOD FOR DETECTING THE REMOVAL OF SENSORS FROM THE SURFACE OF THE SKIN
DISPOSITIF POUR LA FIXATION DE CAPTEURS A LA SURFACE DE LA PEAU ET PROCEDE POUR DETECTER LE RETRAIT DE CES CAPTEURS DE LA SURFACE DE LA PEAU

(30) Priorität: 05.02.1996 DE 19605028
(43) Veröffentlichungstag der Anmeldung: 25.11.1998
(73) Patentinhaber: UFZ-UMWELTFORSCHUNGSZENTRUM LEIPZIG-HALLE GMBH, 04318 Leipzig (DE)
(72) Erfinder: KRUMBIEGEL, Peter, D-04157 Leipzig (DE); ROLLE-KAMPCZYK, Ulrike, D-04347 Leipzig (DE)
(74) Vertreter: Ziebig, Marlene, Dr. Dipl.-Chem.
(86) Internationale Anmeldenummer: EP9700140
(87) Internationale Veröffentlichungsnummer: WO9728772

(56) Entgegenhaltungen:
- US-A- 3 631 617
- US-A- 5 203 327
- US-A- 5 396 901

## Beschreibung

Die Erfindung bezieht sich auf eine Fixiervorrichtung für Sensoren auf der Hautoberfläche und umfaßt ein Nachweisverfahren für das Entfernen solcher Sensoren, mit dem zum Beispiel eine unerlaubte Abnahme des Sensors vom Körper nachweisbar ist.

Vielfach besteht die Notwendigkeit, einen Sensor am menschlichen Körper anzubringen, der bestimmte Signale, die der Körper abgibt (z.B. Töne, Geräusche, Temperaturveränderungen, Flüssigkeitsabsonderungen), oder Signale äußerer Einwirkungen auf den Körper (z.B. durch Schadstoffe, Schall, Hitze, Kälte) ohne Beaufsichtigung über einen längeren Zeitraum aufnimmt und/oder abgibt. Es kann sich dabei um handelsübliche Sensoren handeln, die bestimmte Signale kontinuierlich oder diskontinuierlich registrieren und gegebenenfalls aufzeichnen oder senden, um später ausgewertet zu werden. Solche Sensoren sind im Sinne der Erfindung beispielsweise EKG-Aufzeichnungs- und Analysegeräte, Sensoren zur Aufzeichnung und/oder Auswertung des Pulses (Pulsuhren) oder des Blutdruckes oder Bronchitis-Asthma-Sensoren, wie zum Beispiel in DE 43 38 466 A1 beschrieben.

Üblicherweise werden die Sensoren mit Fixiervorrichtungen wie Fixierstreifen oder Pflastern aus unterschiedlichstem Trägermaterial fest auf der Haut fixiert, so daß sie bis zu einigen Wochen am Körper getragen werden können, und vom Probanden keine Einschränkungen bezüglich Körperreinigung hingenommen werden müssen. Solche Fixiervorrichtungen sind kommerziell in allen denkbaren Formen erhältlich und werden zum Beispiel von der Firma ARBO®-GmbH vertrieben. Weisen die Sensoren zum Beispiel eine runde Form auf, so finden als Fixiervorrichtungen Ringpflaster breite Anwendung. Die Fixierung erfolgt in allen Fällen unabhängig von der Form des Sensors so, daß die üblichen Fixiervorrichtungen Teile des Sensors oder den kompletten Sensor sowie lückenlos eine den Sensor umgebende Hautfläche fest überkleben, wodurch ein enger Hautkontakt der der Hautoberfläche zugewandten Seite des Sensors sowie Wasserdichtigkeit gewährleistet werden.

So ist aus DE-GM 76 28 422 beispielsweise eine Klebevorrichtung zum Applizieren eines Meßgrößenabnehmers (Sensors) für die Funktionsdiagnostik bekannt, die im wesentlichen aus einem Klebering besteht und das Auftreten von Artefakten auch bei heftigster Körperbewegung sicher verhindern soll.

Mit der Weiterentwicklung der Mikroelektronik und Sensortechnik werden die beschriebenen Sensoren zunehmend als juristische Beweismittel für die Beurteilung bestimmter Belastungen einer Person verwendet. In solchen Fällen ist ein Mißbrauch und Betrug durch die betreffende Person nicht auszuschließen, indem zum Beispiel diese Person den Sensor unerlaubt vom eigenen Körper entfernt und entweder einer dritten, zum Beispiel asthmakranken Person überläßt oder im Falle eines Schadstoffsensors an einem besonders schadstoffbelasteten Ort deponiert, der mit dem Aufenthaltsort der Person nicht identisch ist. Kurz vor der vereinbarten Rücknahme des Sensor durch die Amtsperson könnte die zu untersuchende Person den Sensor dann wieder am eigenen Körper anbringen.

Zuverlässige Systeme und Verfahren zum Nachweis des unerlaubten Entfernens von Sensoren von der Haut gibt es bis heute nicht.
Theoretisch denkbare Methoden, wie z.B. die in der Kriminalität in Ausnahmefällen herangezogenen Gen-, Schweiß- und Spurenanalysen, wären für den erfindungsgemäßen Zweck zu aufwendig und teuer bei gleichzeitiger Unsicherheit der Aussage.
So wird zum Beispiel in US 5,203,327 ein Pflaster zur nicht-invasiven Bestimmung von Analyten (zum Beispiel Drogen) in Körperflüssigkeiten beschrieben, das zum Sammeln der ausgeschiedenen Flüssigkeit dient und in dem die Analyten mit immobilisierten spezifischen Bindungspartnern komplexiert und visuell angzeigt werden. Das Pflaster kann bestimmte Marker enthalten, so daß ein Abnehmen des Pflasters von der Haut und eventuelle Verfälschungen der gefundenen Analyten (zum Beispiel durch Verdünnen mit Wasser) angezeigt werden. Für den erfindungsgemäßen Zweck ist diese technische Lösung allerdings zu aufwendig und damit ungeeignet.

Auch die bekannte Methode der Selbstzerstörung von folienartigen Aufklebern bei deren Abschälen von einem starren Untergrund, wie sie z.B. für Autobahn-Vignettenaufkleber oder Preisschilder genutzt wird, ist für das System Pflaster/Haut ungeeignet, weil die Eigenbewegung der Haut zum unkontrollierbaren alsbaldigen Aufreißen der Foliennähte führen könnte, wodurch die von der Fixiervorrichtung geforderte nachhaltige Wasserdichtigkeit und Festigkeit vorzeitig aufgehoben wird.

Der vorliegenden Erfindung liegt die Aufgabe zugrunde, eine Fixiervorrichtung für Hautoberflächen-Sensoren zu entwickeln, mit dem eine Abnahme des Sensors vom Körper nachweisbar ist, sowie ein Verfahren zum Nachweisen des Entfernens von Sensoren von der Hautoberfläche anzugeben.

Die Aufgabe wird mit der Fixiervorrichtung gemäß Anspruch 1 sowie mit dem Nachweisverfahren gemäß Anspruch 9 gelöst.

Bei der Fixiervorrichtung gemäß Anspruch 1 handelt es sich um eine einfache und technisch leicht realisierbare Lösung, mit der zuverlässig ein Abnehmen des Sensors vom Körper der Person, an der personengebunden bestimmte Signale registriert werden sollten, nachträglich nachweisbar ist.

Die erfindungsgemäße Fixiervorrichtung ein Grundpflaster 3 und ein auf die klebende Seite des Grundpflasters 3b aufgebrachtes Erkennungspflaster 4, das eine Ausnehmung aufweist, wodurch zwischen Grundpflaster 3 und Erkennungspflaster 4 ein Hohlraum 5 entsteht, der durch eine geringe Haftung zwischen Grundpflaster 3 und Erkennungspflaster 4 beim Abnehmen des Sensors aufreißt.

Als Trägermaterialien kommen für die erfindungsgemäße Fixiervorrichtung die für solche Fixiervorrichtungen üblichen Materialien in Frage, wie zum Beispiel Schaumträger, mikroporöse Träger, Gewebeträger. Erfindungsgemäß kann die Trägerschicht 4a des Erkennungspflasters aus dem gleichen Trägermaterial wie die des Grundpflasters 3 gefertigt sein. Auch für die Klebeschichten des Grund- und Erkennungspflasters 3b,4b kommen die für solche Fixiervorrichtungen üblichen Kleber in Frage. So kann die Klebeschicht 4b des Erkennungspflasters die gleiche Grundzusammensetzung wie die des Grundpflasters 3b haben und beispielsweise eine Mischung aus Phthalatharz, Polyvinylether und Acrylat-Mischpolymerisat darstellen. In einer bevorzugten Ausgestaltung der Erfindung ist die Trägerschicht 4a des Erkennungspflasters, mit der es ringförmig auf der Klebeschicht 3b des Grundpflaster haftet, aus einem relativ glatten Material, um die Haftung zwischen Grundund Erkennungspflaster gering zu halten. In einer bevorzugten Ausführungsform ist die Haftung zwischen Grund- und Erkennungspflaster zwei- bis zehnfach geringer als zwischen Grund- oder Erkennungspflaster und Haut.

Der Hohlraum 5 des Erkennungspflasters wird in einer bevorzugten Ausführungsform während der Herstellung der erfindungsgemäßen Fixiervorrichtung mit einer Flüssigkeit, einem Feststoff oder einem Gas versehen, so daß beim Zusammenkleben von Grund- und Erkennungspflaster eine Berührung bzw. Haftung der Klebeschicht des Grundpflasters mit dem Innenbereich des Erkennungspflasters verhindert wird. Vorzugsweise kann der Teil des Grundfplasters, der den Hohlraum abschließt, auch ohne Klebeschicht 3b ausgestaltet sein.

Wenn die Klebeschicht 4b des Erkennungspflasters die gleiche Zusammensetzung wie die des Grundpflasters hat, weisen beide Klebeschichten das gleiche Haftvermögen auf der Haut auf. In einer bevorzugten Ausgestaltung der Erfindung weist jedoch die Klebeschicht 4b des Erkennungspflasters eine zwei- bis fünffach höhere Klebekraft auf als die des Grundfplasters 3b. Dies kann z.B. durch einen erhöhten Anteil an Polyacrylat oder Zusatz von Cyanacrylat erreicht werden. In beiden Fällen ist jedoch die Klebekraft zwischen Grund- und Erkennungspflaster geringer als zwischen Erkennungspflaster oder Grundpflaster und Haut. Dadurch bleibt beim Abreißen des Grundpflasters das Erkennungspflaster auf der Haut haften und der aufgerissene Hohlraum 5 dient dem Nachweis des Entfernens des Sensors.

In einer weiteren Ausgestaltung der Erfindung weist der den Hohlraum 5 abdeckende Teil des Grundpflasters keine Klebeschicht 3b auf, und die Trägerschicht 3a dieses Teils des Grundpflasters besteht aus durchsichtigem Material, vorzugsweise aus Polyethylen oder Polypropylen.

Dadurch kann die Amtsperson schon ohne Abnahme des Fixiersystems dessen Unverletztheit kontrollieren.

In einer weiteren bevorzugten Ausführungsform beinhaltet der Hohlraum 5 zwischen Grund- und Erkennungspflaster eine Farbstoff-, Feststoff- oder Geruchsmarkierung, die dem Nachweis des unerlaubten Entfernens dienen soll, gemäß den Ansprüchen 3 bis 6.

Die Größe des Hohlraumes 5 ist durch die Ausnehmung im Trägermaterial 4a des Erkennungspflasters beliebig gestaltbar. Beinhaltet der Hohlraum 5 eine Farbstofflösung als Markierung, so ist bereits ein sehr kleines Volumen ausreichend. Beim Lösen der Fixiervorrichtung vom Körper wird der Hohlraum 5 aufgerissen und der austretende Farbstoff verfärbt das benachbarte Trägermaterial (zum Beispiel Gewebe) und/oder die darunterliegende Hautfläche.

Erfindungsgemäß werden bevorzugt Leukofarbstoffe zur Markierung des Hohlraumes 5 eingesetzt, beispielsweise Leukomalachitgrün oder Leukokristallviolett (Lieferer: Fa. Aldrich).

Wird der Sensor von der Amtsperson abgenommen, so weist eine Haut- und/oder Pflasterverfärbung darauf hin, daß der Sensor bereits entfernt wurde. Wird hingegen die Farbstofflösung erst jetzt freigesetzt, so ist der Sensor zwischenzeitlich nicht entfernt worden.

In einer weiteren Ausgestaltung der Erfindung gemäß Anspruch 5 kann der Hohlraum 5 auch durch einen charakteristisch gefärbten Feststoff und/oder einen Feststoff mit charakteristischer räumlicher Struktur markiert sein, dessen Fehlen ein zwischenzeitliches unbefugtes Entfernen der Fixiervorrichtung signalisiert. Vorzugsweise kommen hier Polyethylengranula oder gefärbte Bleiglaskügelchen zur Anwendung. Erfindungsgemäß kommen solche Feststoffe zum Einsatz, die dem durchschnittlichen Personenkreis nicht zur Verfügung stehen, so daß ein Mißbrauch durch Auffüllen und Wiederanbringen ausgeschlossen ist.

In einer weiteren Ausgestaltung der Erfindung kann der Hohlraum 5 der Fixiervorrichtung auch eine Geruchsmarkierung über einen Duftstoff oder ein charakteristisch riechendes Gas (wie zum Beispiel Schwefelwasserstoff) beinhalten, so daß das Fehlen des entsprechenden charakteristischen Geruches bei der Abnahme des Sensors durch die Amtsperson das unbefugte Entfernen anzeigt.

Nachfolgend ist die Erfindung anhand von Ausführungsbeispielen näher erläutert.

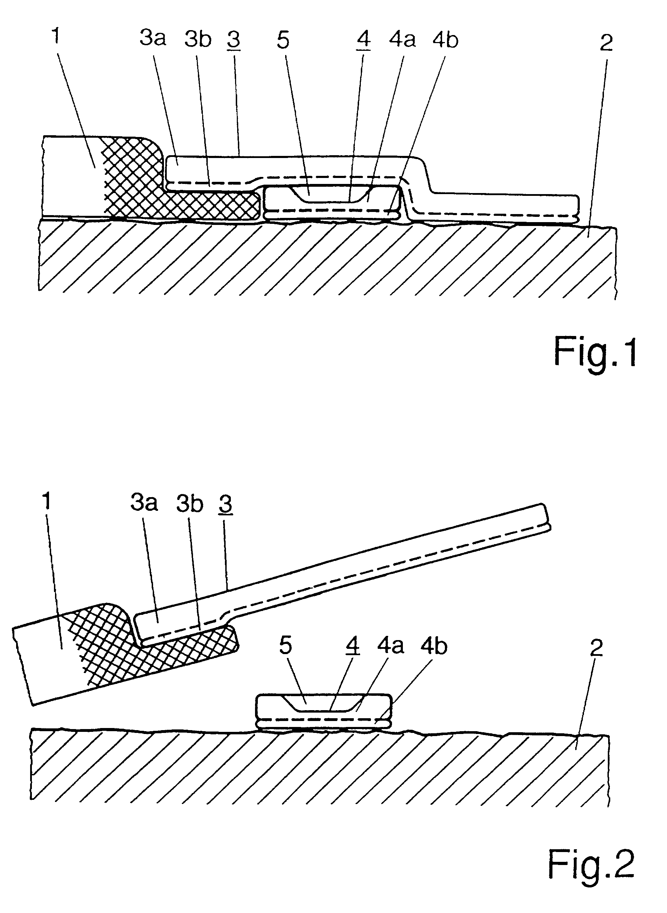

Bevorzugte Ausführungsvarianten der Erfindung sind in den Fig. 1 und 2 dargestellt, wobei
Fig. 1 den Querschnitt der Fixiervorrichtung im aufgeklebten Zustand zeigt und
Fig.2 den Querschnitt der Fixiervorrichtung nach Entfernen des Sensors von der Haut zeigt.

### Beispiel 1

Ein Ringpflaster 3 mit 8 cm äußerem Durchmesser und 3,5 cm innerem Durchmesser, das zum Festkleben eines Sensors von der Größe einer Armbanduhr dienen soll, enthält integriert, d.h. durch Aufkleben eines Erkennungspflasters 4 von 1 cm Durchmesser während des Herstellungsprozesses, eine flache runde Kapsel von 1 mm innerer Höhe und 5 mm innerem Durchmesser, die beim Ablösen des Ringpflasters 3 von der Haut zwangsweise dadurch geöffnet wird, daß nur das Erkennungspflaster 4 auf der Haut haften bleibt. Dadurch werden die wenigen Tropfen eines beim Herstellungsprozess in die Kapsel injizierten Farbstoffes, wie z.B. einer 10%igen wäßrigen Eosinlösung, freigesetzt. Der Farbstoff verfärbt das benachbarte Pflaster und die Haut. Das auf der Haut haften gebliebene Erkennungspflaster 4 wird abschließend (von der Amtsperson) ebenso abgelöst wie zuvor das Grundpflaster 3.

### Beispiel 2

Es wird wie in Beispiel 1 verfahren, jedoch der Leukofarbstoff Leukomalachitgrün verwendet. Bei dieser erfindungsgemäßen Variante tritt die Hautfärbung erst nach Sauerstoffzutritt auf.

### Bezugszeichenliste

- 1: Sensor
- 2: Haut
- 3: Grundpflaster
- 3a: Trägerschicht des Grundpflasters
- 3b: Klebeschicht des Grundpflasters
- 4: Erkennungspflaster
- 4a: Trägerschicht des Erkennungspflasters
- 4b: Klebeschicht des Erkennungspflasters
- 5: Hohlraum im Erkennungspflaster

## Patentansprüche

1. Fixiervorrichtung für Sensoren auf der Hautoberfläche, die den Sensor (1) fest auf der Haut (2) fixiert,
**dadurch gekennzeichnet,**
**daß** die Fixiervorrichtung ein Grundpflaster (3), bestehend aus einer Trägerschicht (3a) und einer Klebeschicht (3b) aufweist, wobei an der Klebeschicht (3b) des Grundpflasters (3) ein Erkennungspflaster (4), das in der Trägerschicht (4a) eine Ausnehmung aufweist, so angebracht ist, daß zwischen Grund- und Erkennungspflaster ein Hohlraum (5) vorhanden ist, wobei das Haftvermögen zwischen der Trägerschicht des Erkennungspflasters (4a) und der Klebeschicht (3b) des Grundpflasters (3) geringer ist als das Haftvermögen von Grundpflaster (3) und Erkennungspflaster (4) zur Haut (2).

2. Fixiervorrichtung nach Anspruch 1,
**dadurch gekennzeichnet,**
**daß** der den Hohlraum (5) abdeckende Teil des Grundpflasters (3) keine Klebeschicht (3b) aufweist und die Trägerschicht (3a) dieses Teils des Grundpflasters (3) aus durchsichtigem Material besteht, vorzugsweise aus Polyethylen oder Polypropylen.

3. Fixiervorrichtung nach Anspruch 1 oder 2,
**dadurch gekennzeichnet,**
**daß** im Hohlraum (5) eine Farbstofflösung, ein Feststoff, ein Duftstoff oder ein Gas eingeschlossen sind.

4. Fixiervorrichtung nach Anspruch 3,
**dadurch gekennzeichnet,**
**daß** der Farbstoff ein Leukofarbstoff ist

5. Fixiervorrichtung nach Anspruch 3,
**dadurch gekennzeichnet,**
**daß** die Festsubstanz eine charakteristische Farbe und/oder räumliche Struktur aufweist.

6. Fixiervorrichtung nach Anspruch 3,
**dadurch gekennzeichnet,**
**daß** das Gas einen charakteristischen Geruch aufweist.

7. Fixiervorrichtung nach den Ansprüche 1 bis 3,
**dadurch gekennzeichnet,**
**daß** das Haftvermögen von Grundpflaster (3) und Erkennungspflaster (4) zur Haut (2) gleich groß ist.

8. Fixiervorrichtung nach den Ansprüchen 1 bis 3,
**dadurch gekennzeichnet,**
**daß** das Haftvermögen des Erkennungspflasters (4) zur Haut (2) größer ist als das Haftvermögen des Grundpflasters (3) zur Haut (2).

9. Verfahren zum Nachweis des Entfernens von Sensoren von der Hautoberfläche unter Verwendung einer Fixiervorrichtung nach einem der Ansprüche 1 bis 8,
**dadurch gekennzeichnet,**
**daß** beim Entfernen eines mittels der Fixiervorrichtung fest auf der Haut (2) fixierten Sensors (1) das Grundpflaster (3) von der Haut (2) gelöst wird, das Erkennungspflaster (4) mit seiner Klebeschicht (4b) an der Haut (2) haften bleibt, wodurch der Hohlraum (5) aufreißt und mittels des aufgerissenen Hohlraumes der Nachweis des Entfernens des Sensors geführt wird.

10. Verfahren nach Anspruch 9,
**dadurch gekennzeichnet,**
**daß** der Nachweis des Entfernens mittels einer Farbstofflösung, eines Feststoffes, eines Duftstoffes oder eines Gases geführt wird, die im Hohlraum (5) vorhanden sind und beim Ablösen des Sensors (1) von der Haut (2) aus diesem Hohlraum austreten.

## Claims

1. A device for attaching sensors to the surface of the skin tightly fixing the sensor (1) to the skin (2),
**characterized in that**
said attachment device comprises a basic plaster (3) consisting of a supporting layer (3a) and an adhesive layer (3b), wherein a detection plaster (4) comprising a recess in its supporting layer (4a) is attached to said adhesive layer (3b) of said basic plaster (3) in such a manner to provide a hollow space (5) between said basic and detection plasters and wherein the adhesion capability between the supporting layer of said detection plaster (4a) and the adhesive layer (3b) of said basic plaster (3) is smaller than the adhesion capability of said basic plaster (3) and said detection plaster (4) to the skin (2).

2. Attachment device according to claim 1,
**characterized in that**
the portion of said basic plaster (3) covering said hollow space (5) does not comprise an adhesive layer (3b), while the supporting layer (3a) of said portion of said basic plaster (3) consists of transparent material, preferably polyethylene or polypropylene.

3. Attachment device according to claim 1 or 2,
**characterized in that**
said hollow space (5) contains a dye solution, a solid matter, an odourised substance or a gas.

4. Attachment device according to claim 3,
**characterized in that**
the dye is a leuco dye.

5. Attachment device according to claim 3,
**characterized in that**
the solid matter has a characteristic colour and/or spatial structure.

6. Attachment device according to claim 3,
**characterized in that**
the gas has a characteristic odour.

7. Attachment device according to claims 1 through 3,
**characterized in that**
both of said basic plaster (3) and said detection plaster (4) have the same adhesion capability to the skin (2).

8. Attachment device according to claims 1 through 3,
**characterized in that**
the adhesion capability of said detection plaster (4) to the skin (2) is greater than the adhesion capability of said basic plaster (3) to the skin (2).

9. A method for proving the removal of sensors from the surface of the skin using an attachment device according to any of claims 1 through 8,
**characterized in that**
during the removal of a sensor (1) being tightly attached to the skin (2) by means of the attachment device, said basic plaster (3) is removed from the skin (2) while said detection plaster (4) remains sticking to the skin (2) with its adhesive layer (4b), causing said hollow space (5) to tear open and thus proving the removal of the sensor.

10. Method according to claim 9,
**characterized in that**
the removal is proved by means of a dye solution, a solid matter, an odourised substance, or a gas being present inside said hollow space (5) and which escape from said hollow space during the removal of the sensor (1) from the skin (2).

## Revendications

1. Dispositif de fixation de senseurs sur la surface de la peau, assurant une fixation ferme du senseur (1) sur la surface de la peau (2),
**caractérisé en ce que**
le système de fixation présente un pansement de base (3), consistant en une couche support (3a) et une couche collante (3b), un pansement de détection (4) avec un évidement dans la couche support (4a) étant appliqué sur la couche collante (3b) du pansement de base (3), de manière à créer une cavité (5) entre le pansement de base et le pansement de détection, l'adhérence entre la couche support du pansement de détection (4a) et la couche collante (3b) du pansement de base (3) étant moindre que celle du pansement de base (3) et du pansement de détection (4) sur la peau (2).

2. Dispositif de fixation selon revendication 1,
**caractérisé en ce que**
la partie du pansement de base (3) recouvrant la cavité (5) ne présente pas de couche collante (3b) et que la couche support (3a) de cette partie du pansement de base (3) consiste en un matériau transparent, préférentiellement du polyéthylène ou du polypropylène

3. Dispositif de fixation selon revendication 1 ou 2,
**caractérisé en ce que**
la cavité (5) contient une solution colorante, une substance solide, une substance odorante ou un gaz.

4. Dispositif de fixation selon revendication 3,
**caractérisé en ce que**
le colorant est un leuco-colorant.

5. Dispositif de fixation selon revendication 3,
**caractérisé en ce que**
la substance solide présente une couleur et/ou une structure spatiale caractéristiques.

6. Dispositif de fixation selon revendication 3,
**caractérisé en ce que**
le gaz présente une odeur caractéristique.

7. Dispositif de fixation selon revendications 1 à 3,
**caractérisé en ce que**
l'adhérence du pansement de base (3) est identique à celle du pansement de détection (4) sur la peau (2).

8. Dispositif de fixation selon revendications 1 à 3,
**caractérisé en ce que**
l'adhérence du pansement de détection (4) sur la peau (2) est supérieure à celle du pansement de base (3) sur la peau (2).

9. Procédé pour constater le retrait de senseurs de la surface de la peau si un système de fixation selon l'une des revendications 1 à 8 est utilisé,
**caractérisé en ce que**
lors du retrait d'un senseur (1) fixé sur la peau (2) au moyen du système de fixation, le pansement de base (3) est détaché de la peau (2), le pansement de détection (4) reste adhérent à la peau (2) par sa couche collante (4b), la cavité (5) étant alors ouverte, la preuve du retrait du senseur étant apportée par l'ouverture de la cavité.

10. Procédé selon revendication 9,
**caractérisé en ce que**
la preuve du retrait sera apportée par une solution colorante, une substance solide, une substance odorante ou un gaz, présent(e) dans la cavité (5) et se dégageant hors de celle-ci lors du détachement du senseur (1) de la peau (2).
